# EUROPEAN PATENT APPLICATION

(11) **EP 4 591 876 A1**
(43) Date of publication of application: **30.07.2025**
(21) Application number: 24382064.4
(22) Date of filing: 25.01.2024
(51) Int. Cl.: A61K 38/44, A23L 33/105, A61P 25/20, A61P 25/28

(54) **DIAMINE OXIDASE (DAO) FOR USE IN THE TREATMENT AND/OR PREVENTION OF SLEEP AND CIRCADIAN RHYTHM DISORDERS, AND COGNITIVE IMPAIRMENT ASSOCIATED THERETO**

(71) Applicant: DR Healthcare S.L.U., 08017 Barcelona (ES)
(72) Inventor: DUELO RIU, Juan José, Barcelona (ES); DE LECEA FLORES DE LEMUS, Carlos, Barcelona (ES); TINTORÉ GAZULLA, Maria, Barcelona (ES); CUÑÉ CASTELLANA, Jordi, Rubí (ES)
(74) Representative: Ponti & Partners, S.L.P

(57) **Abstract**

The present invention relates to diamine oxidase (DAO) for use in the treatment and/or prevention of sleep and circadian rhythm disorders, and cognitive impairment associated thereto.

## Description

### Field of the invention

The present invention relates to diamine oxidase (DAO) for use in the treatment and/or prevention of sleep and circadian rhythm disorders, and cognitive impairment associated thereto.

### Background

Sleep disorders are widely prevalent world-wide. In order to organize said sleep disorders several classifications have been suggested. In one of them categories of sleep disorders have been identified as: (i) insomnia, (ii) hypersomnia, (iii) parasomnia, (iv) circadian rhythm sleep-wake disorders, (v) sleep-related breathing disorders, and (vi) sleep movement disorders. Under another classification scheme, ten broad primary categories of sleep disorders have been identified: (1) insomnia disorder, (2) hypersomnolence disorder, (3) narcolepsy, (4) breathing-related sleep disorders, (5) circadian rhythm sleep-wake disorders, (6) non-rapid eye movement ("NREM") sleep arousal disorders, (7) nightmare disorder and abnormal dreams, (8) rapid eye movement sleep behaviour disorder, (9) restless leg syndrome, and (10) substance/medication-induced sleep disorder. The most popular and with a higher prevalence in the population is perhaps insomnia.

According to the International Classification of Sleep Disorders - Third Edition (ICSD-3), insomnia is defined as a complaint of trouble initiating or maintaining sleep which is associated with daytime consequences and is not attributable to environmental circumstances or inadequate opportunity to sleep. In industrialized countries, about 6% of the adults suffer from chronic insomnia (Ohayon MM. Epidemiology of insomnia: what we know and what we still need to learn. Sleep Med Rev. 2002;6(2):97-111. doi:10.1053/smrv.2002.0186; Riemann D, Baglioni C, Bassetti C, et al. European guideline for the diagnosis and treatment of insomnia. J Sleep Res. 2017;26(6):675-700. doi:10.1111/jsr.12594). Particularly, insomnia's prevalence varies largely among European countries, from a minimum of 5.7% in Germany to a maximum of 19% in France and being of 6.9% in the Spanish population (Riemann et al., see above). Several studies have shown that insomnia is a risk factor for several diseases (Riemann et al., see above), thus having a better comprehension of this disorder may contribute to personalize its evaluation and management, as well as to prevent other related diseases.

Pharmaceutical drugs used for the management of insomnia may cause important side effects, in addition to having potentially dangerous interactions with other medications. In addition, they are prone for abuse and dependence, and they should be used under medical supervision and as a rescue medication and not for chronic use (Madari S, Golebiowski R, Mansukhani MP, Kolla BP. Pharmacological Management of Insomnia. Neurotherapeutics. 2021 Jan;18(1):44-52. doi: 10.1007/s13311-021-01010-z. Epub 2021 Feb 1. PMID: 33527255; PMCID: PMC8116439. Pharmacological Management of insomnia - PMC (nih.gov)). Some examples are:
∘ Non-Benzodiazepine Receptor Agonists such as Zolpidem, etc: Most commonly associated side effects with the use of zolpidem are diarrhea, nausea, dizziness, somnolence, and visual disorders. All nBBRAs are linked with complex behaviors occurring out of sleep but zolpidem seems to be especially associated with this side effect
o benzodiazepines are associated with ataxia, dizziness, lethargy, sedation, somnolence, apnea, and blurred vision. Although there are reports indicating increased risk of anterograde amnesia on triazolam, this can likely occur with other benzodiazepines as well
∘ barbiturates:
   ▪ potential abuse and dependence,
   ▪ significant interactions with alcohol and other CNS depressants
   ▪ very narrow therapeutical windows, in some cases (chloral hydrate) potentially dangerous following overdose
∘ orexin receptor antagonists:
   ▪ suvorexant: sleepiness/fatigue, abnormal dreams, dry mouth, and diarrhea. There is a concern for increased risk of suicidal ideation with the higher dose regimen
   ▪ Lemborexant: headache, sleepiness, and sleep paralysis.
o Sedative antidepressants: headache and somnolence, trazodone is associated with deleterious effects on short-term memory and balance, and in rare occasions, with priapism
o Antihistamins: daytime grogginess, anticholinergic side effects

In addition to the above limitations for pharmaceutical drugs, when used in dietary supplements some products also have the following side effects:
∘ Melatonin:
   ▪ Main side effect is daytime somnolence
   ▪ Dose is calculated in an empiric way, since there are no clear biomarkers for measuring it
   ▪ Melatonin is a hormone, a wrong or inadequate dose can induce a negative feedback or dysregulation
   ▪ wide variation of content compared to that advertised in the label,
∘ botanical extracts:
   low quality of evidence and questionable efficacy in several cases (Leach MJ, Page AT. Herbal medicine for insomnia: A systematic review and meta-analysis. Sleep Med Rev. 2015;24:1-12. Herbal medicine for insomnia: A systematic review and meta-analysis - PubMed (nih.gov)) In particular, valerian root is the most studied extract commercialized as dietary supplement for sleep management. However, a meta-analysis of 5 trials did not find any significant difference between valerian root and placebo in sleep-related outcomes.
   ▪ botanical extract's limitations include that unless there is a correct standardization of their active compounds, which is sometimes challenging, it is very difficult to adjust doses or ensure quality
   ▪ botanical extracts are highly reactive and interact with other active ingredients

Accordingly, the problem to be solved by the present invention is to find an alternative way for treating, improving and preventing sleep and circadian rhythm disorders, and cognitive impairment associated thereto. The present invention addresses this problem by employing DAO.

**Diamine oxidase** (DAO) is an enzyme encoded by the *AOC1* gene (OMIM#104610), located on chromosome 7 (7q-34-36). It is a secretory protein stored in vesicular structures of plasma membrane. It is responsible for the degradation of extracellular histamine. In mammals, DAO is expressed mainly in the small intestine and is located in the intestinal villi. DAO encoding gene polymorphisms have been analyzed in depth and more than 50 non-synonymous single nucleotide polymorphisms (SNPs) that can alter its activity have been identified. The three more relevant SNPs affecting Caucasian individuals that lead to a reduction of its enzymatic activity are: c.47C>T (rs10156191), c.995C>T (rs1049742), c.1990C>G (rs1049793) (Ayuso P, Garcia-Martin E, Martinez C, Agúndez JA. Genetic variability of human diamine oxidase: occurrence of three nonsynonymous polymorphisms and study of their effect on serum enzyme activity. Pharmacogenet Genomics. 2007;17(9): 687-693. doi:10.1097/FPC.0b013e328012b8e4; Garcia-Martin E, Ayuso P, Martinez C, Agúndez JA. Improved analytical sensitivity reveals the occurrence of gender-related variability in diamine oxidase enzyme activity in healthy individuals. Clin Biochem. 2007;40(16-17):1339-1341. doi:10.1016/j.clinbiochem.2007.07.019). Ayuso et al., described the following frequencies for these AOC1 variants (95% confidence interval) among Spanish Caucasian individuals: rs10156191, 25.4% (20.16-30.58), rs1049742, 6.3% (3.42-9.26) and rs1049793, 30.6% (25.1-36.1).

### Summary of the invention

The present invention relates to diamine oxidase (DAO) for use in the treatment and/or prevention of sleep and circadian rhythm disorders, and cognitive impairment associated thereto.

### Detailed description

The present invention relates to diamine oxidase (DAO) for use in the treatment and/or prevention of sleep and circadian rhythm disorders, and cognitive impairment associated thereto. Preferably, said sleep and circadian rhythm disorders are insomnia and/or abnormal dreams.

**"Sleep and circadian rhythm disorders"** in the context of the present invention relates to the disorders mentioned above according to a first classification: (i) insomnia, (ii) hypersomnia, (iii) parasomnia, (iv) circadian rhythm sleep-wake disorders, (v) sleep-related breathing disorders, and (vi) sleep movement disorders; or according to another classification scheme: (1) insomnia disorder, (2) hypersomnolence disorder, (3) narcolepsy, (4) breathing-related sleep disorders, (5) circadian rhythm sleep-wake disorders, (6) non-rapid eye movement ("NREM") sleep arousal disorders, (7) nightmare disorder or abnormal dreams, (8) rapid eye movement sleep behaviour disorder, (9) restless leg syndrome, and (10) substance/medication-induced sleep disorder. The most popular and with a higher prevalence in the population is insomnia and abnormal dreams.

**"Circadian rhythms"** are the physical, mental, and behavioral changes an organism experiences over a 24-hour cycle. Light and darkness have the biggest influence on circadian rhythms, but food intake, stress, physical activity, social environment, pharmacological and/or hormonal treatments and temperature also affect them.

**"Cognitive impairment associated to Sleep and circadian rhythm disorders"** are a group of daytime symptoms experienced as a consequence of poor sleep quality, including attention, memory, executive function and to a lesser degree, concentration (Brownlow JA, Miller KE, Gehrman PR. Insomnia and Cognitive Performance. Sleep Med Clin. 2020 Mar;15(1):71-76. doi: 10.1016/j.jsmc.2019.10.002. Epub 2019 Nov 27. PMID: 32005351; PMCID: PMC7000136).

**"DAO"** is the abbreviation used to designate the enzyme diamine oxidase responsible for the catalysis of the oxidative deamination of biogenic amines, for example of the primary amine group of histamine to give imidazole acetaldehyde. DAO in the present invention may have different origin, e.g. a non-plant origin, plant origin or biotechnological origin. "Non-plant origin" means any DAO that is not obtained from plants but from animal organisms or other non-plant organisms. Thus, this definition includes all DAO isolated from living creatures that are not plants. "Plant origin" means any DAO obtained from plant organisms. "Biotechnological origin" means any DAO prepared from recombinant cell cultures or in non-plant organisms of any type after isolating the DNA for DAO.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

It should be noted that the term "approximately" or "about" applied to the values used earlier and later in this document includes a margin of error of ± 5 %, such as, for example, ± 4 %, ± 3 %, ± 2 %, ± 1 %.

As used herein, the term "treat" or "treatment" or "treating" and its cognates refer to an amelioration/improvement of at least one discernible symptom of sleep and circadian rhythm disorders, and cognitive impairment associated thereto, or the questionaries based thereon (See examples). In another embodiment, "treat" refers to an amelioration/improvement of at least one measurable physical parameter, not necessarily discernible by the patient, in another embodiment, "treat" or "treatment" or "treating" refer to inhibiting the progression of at least one discernible symptom of sleep and circadian rhythm disorders, and cognitive impairment associated thereto, either physically (e.g., stabilization of a discernible symptom), physiologically (e.g., stabilization of a physical parameter), or both. In another embodiment, "treat" or "treatment" or "treating" refer to slowing the progression or reversing the progression of at least one discernible symptom of sleep and circadian rhythm disorders, and cognitive impairment associated thereto. As used herein, "prevent" or "prevention" and its cognates refer to delaying the onset or reducing the risk of acquiring or recurring at least one discernible symptom of sleep and circadian rhythm disorders, and cognitive impairment associated thereto. The general terms "prevent" or "prevention" also include the prevention of recurrence in subjects that have had previous episodes of sleep and circadian rhythm disorders, and cognitive impairment associated thereto.

As used herein, the term "preventing and/or treating" includes "preventing and treating" and "preventing or treating".

As used herein the term "supplementation" includes "dietary management", "administration of food supplements, dietary supplements or food for special medical purposes".

In another embodiment, said diamine oxidase (DAO) is combined with at least one another active ingredient. Said at least another active ingredient may be a compound selected from a botanical extract, a pharmaceutical drug for treating sleep and circadian rhythm disorders and a combination thereof.

Said botanical extract is selected from Crocus sativus L., Melissa officinalis, Melissa officinalis folium, Avena sativum, Crataegus laevigata, Lavandula officinalis flos, Lavandula angustifolia, Melilotus officinalis, Papaver rhoeas, Valeriana officinalis, Valeriana officinalis radix, Bigaradier feuille, Valerian- Humulus lupulus combination, Tilia species flos, Tilia platyphyllos, Aloysia triphylla, Anethum graveolens, Centaurium erythrea, Galium odoratum, Humulus lupulus, Humulus lupulus strobuli, Eschscholtzia californica, Asparagus racemosus root, Convolvulus pluricaulis, combination of valerian root, lemon balm leaves and chamomile flowers, Sida cordifolia, Withania somnifera, Bitter orange, Hypericum perforatum-Herba hyperici plant-St. John Wort, Salix alba-Buds-White Willow, Thuja orientalis-seeds-Arbor vitae, Passiflora incarnata herba, Melilotus officinalis herba, Majorana hortensis herba, Crataegus monogyna folium cum flore, Matricaria chamomilla flos, Magnesium lactate, Hippophae rhamnoides, sea-buckthorns, fruit vitamin B6-PVP-aerosil-starch-tablettose-avicel-stearic acid, Mentha piperita, Ballota nigra L., xanthohumol enriched hop extract, Hop extract containing xanthohumol, xanthohumol, Hop extract, Artemisia dracunculus, Grindelia robusta nutt., Marrubium vulgare I., Ruta graveolens I., Tabebuia avellanedae, Passiflora incarnata, passion flower, alcohol water Pi-nipagin-nipasol, Ziziphi Spinosae, Panax ginseng, Panax quinquefolius, Poria cocos, Polygala tenuifolia, Ganoderma lucidum, Schisandra chinensis, Platycladus orientalis, Acanthopanax senticosus, Fallopia multifloraVeronica officinalis, Calluna vulgaris, Filipendula ulmaria, Bacopa montiera, Basilic, Cassia italica f.w. andr., Epilobium angustifolium I., Primula veris I. em.heids, Tanacetum parthenium, and a combination thereof.

Said pharmaceutical drug for treating sleep and circadian rhythm disorders is selected from sedating antidepressants, sedating antipsychotics, benzodiazepine receptor agonists, non-benzodiazepine benzodiazepine receptor agonists (nBBRAs), barbiturates, inhibitors of orexin and hypocretin, doxepin, melatonin, agonists of melatonin receptors, and a combination thereof.

In a preferred embodiment, said diamine oxidase is included in a pharmaceutical composition. In a further preferred embodiment, said pharmaceutical composition further comprises a pharmaceutically acceptable vehicle, adjuvant, diluent or excipient, and optionally at least another active ingredient. Said pharmaceutically acceptable vehicle, adjuvant, diluent or excipient can be any of preserving agents, fillers, disintegrating agents, humidifying agents, emulsifying agents, suspending agents, solvents, dispersion media, coatings, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Said at least another active ingredient can be any of those mentioned above.

In another preferred embodiment, said prevention and/or treatment comprises topically, transdermally or orally, preferably orally, administering said diamine oxidase or the pharmaceutical composition comprising thereof.

In a preferred embodiment, the DAO or the pharmaceutical composition comprising thereof is administered topically. The dosage form may be selected from liquid in spray, cream, lotion, ointment, and liposomes.

In another preferred embodiment, the DAO or the pharmaceutical composition comprising thereof is administered transdermally. The dosage form may be a patch.

In a more preferred embodiment, the DAO or the pharmaceutical composition comprising thereof is administered orally. The dosage form may be selected from a tablet, capsule, powder, microcapsule, minicapsule, nanocapsule, sachet, liposome, and drink. In another preferred embodiment, said dosage forms may have gastric protection for protecting the DAO from gastric acidity. The enteric coating layer that coats the various forms rapidly disintegrates or dissolves in a neutral or alkaline medium. The enteric coating layers may contain pharmaceutically acceptable plasticisers to obtain the desired mechanical properties of flexibility and hardness. These plasticisers can be, for example, triacetin, citric acid esters, phthalic acid esters, cetyl alcohol, polyethylene glycols, polysorbates or other plasticisers.

The presence of the gastric protection would depend on the dosage form. For example, for topical administration this gastric protection is not necessary. Accordingly, the present invention encompasses dosage forms with and without gastric protection.

The above mentioned dosage forms may also contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavouring agent such as peppermint, or methyl salicylate. In addition, dosage unit forms can contain various other materials that modify the physical form of the dosage unit, for example, coatings of sugar, shellac, or enteric agents. Other oral dosage forms like syrup or elixir may contain sweetening agents, preservatives, dyes, colourings, and flavourings. In addition, the active compounds may be incorporated into fast dissolve, modified-release or sustained-release preparations and formulations, and wherein such sustained-release formulations are preferably bimodal.

DAO or the compositions according to the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

DAO can also be preferably mixed with pharmaceutically acceptable substances such as salts of phosphoric acid and sodium, potassium, calcium, magnesium and aluminium, carbonic acid, citric acid or other suitably weak organic and inorganic acids; a co-precipitate of aluminium hydroxide/sodium bicarbonate; substances normally used in anti-acid preparations such as hydroxides of aluminium, calcium and magnesium; magnesium oxide or compound substances such as Al₂O₃.6MgO.CO₂.12H₂O, (Mg₆Al₂(OH)₁₆CO₃.4H₂O, MgO.Al₂O₃.2SiO₂.nH₂O or similar compounds; pH buffering substances such as tris(hydroxymethyl)aminomethane, basic amino acids and their salts or other pharmaceutically acceptable pH buffering substances.

In another preferred embodiment, said diamine oxidase is included in a functional food or a dietary supplement (for example, refreshment, foodstuffs, , chewing gums, sweets or candy), alone or optionally combined with at least one another active ingredient as mentioned above. In other words, said diamine oxidase is included in food matrices providing them with the enzymatic activity (functional food) and helping in reducing their content in biogenic amines. Diamine oxidase could be incorporated in such food matrices during the process of elaboration (processing aid) or as an ingredient declared in the composition of the finished product. Said diamine oxidase included in a functional food or a dietary supplement can be present with any media or agent/compound compatible with the diamine oxidase and the optional at least one another active ingredient. Examples of such media or agent/compound are preserving agents, fillers, disintegrating agents, wetting agents, emulsifying agents, suspending agents, solvents, dispersion media, coatings, isotonic and absorption delaying agents and the like. Said functional food or dietary supplement is preferably orally administered.

"Alone" is meant to be as the unique active ingredient but including any acceptable vehicle, adjuvant, diluent or excipient suitable for such functional food or dietary supplement.

In another embodiment, the pharmaceutical composition, the dietary supplement or the functional food comprising DAO is administered before or with meals and/or once per day, preferably before going to sleep, or once per day, by adapting to the circadian rhythm of endogenous secretion of DAO, or once per day, by adapting to the sleep-wake cycles of the subject.

In another preferred embodiment, said diamine oxidase or the pharmaceutical composition comprising thereof is a cosmetical or medical device or is included in powder, semi-solid, semi-liquid or liquid form in a delivery system which is a medical device which facilitates the administration of the enzyme. Said diamine oxidase is present alone or optionally combined with at least one another active ingredient as mentioned above. Said diamine oxidase as a cosmetical or medical device or included in the cosmetical or medical device can be present with any media or agent/compound compatible with the diamine oxidase and the optional at least one another active ingredient. Examples of such media or agent/compound are preserving agents, fillers, disintegrating agents, wetting agents, emulsifying agents, suspending agents, solvents, dispersion media, coatings, isotonic and absorption delaying agents and the like.

The present disclosure also relates to a method of preventing and/or treating sleep and circadian rhythm disorders, and cognitive impairment associated thereto, according to the embodiments included herein, comprising the step of administering a therapeutically effective amount or dose of DAO according to the embodiments included herein. The terms "therapeutically effective dose" and "therapeutically effective amount" are used to refer to an amount of the subject compound that results in prevention, delay of onset of symptoms, or amelioration of symptoms in the context of the present invention. A therapeutically effective amount, as well as a therapeutically effective frequency of administration, can be determined by methods known in the art and discussed below.

It is noted that any of the embodiments disclosed herein can be taken alone or combined with any other embodiment disclosed herein unless the context specifies otherwise. In other words, for example, a preferred option of a defined feature can be combined with a more or less preferred option of another feature.

The invention will be now illustrated with the following examples, which do not intend to limit its scope.

### EXAMPLES

### EXAMPLE 1 Prevalence of DAO (diamine oxidase) deficiency in patients with insomnia symptoms.

**Main objective:** Estimate the prevalence of DAO deficiency of genetic origin in patients who report symptoms of insomnia.

**Secondary objective:** Correlate DAO deficiency with the insomnia symptoms, frequency of appearance of insomnia symptoms and daytime repercussions.

**Procedure:** All patients treated at AdSalutem Sleep Institute, and who report symptoms of insomnia, were proposed to participate in the study. After having read the patient information sheet and signing the informed consent, all the information necessary for the study was collected and a sample of oral mucosa was obtained. The 4N6 FLOQSwabs^{®} Active Drying System with standard head was used to collect the sample. The swab of the device was introduced into the patient's oral cavity above the tongue and held in position for ten seconds to impregnate the swab with saliva. The swab was then moved between the cheek and the gum. Using the other hand, apply light pressure to the cheek and rubbed the swab back and forth ten times. This was repeated in the other oral cavity. Echevarne Laboratory was in charge of processing and analyzing the samples. No adverse effects were described from the extraction of a sample from the oral mucosa.

Finally, Laboratorio Echevarne issued an individual report on the genotype of each patient and the implications of that genotype on the activity of the DAO. Patient samples were destroyed by the Echevarne Laboratory several weeks after delivering the results. AdSalutem ultimately sent said results report to each of the study participants.

### Study population and selection criteria :

The study subjects (n = 126) are patients who report symptoms of insomnia and who attended the AdSalutem sleep specialist's office.

### Selection criteria

### Inclusion criteria:

- Adult.
- Both genders.
- With the ability to understand and sign the informed consent.
- They report one or more symptoms of insomnia, these being:
   ▪ Difficulty initiating sleep.
   ▪ Difficulty maintaining sleep.
   ▪ Waking up early with inability to go back to sleep
- Explicit acceptance of participation through signing the informed consent.

### Exclusion criteria:

- That the symptoms of insomnia cannot be justified by the existence of another sleep pathology: e.g. e.g., narcolepsy, circadian rhythm disturbances, parasomnia, etc.

### Determination of sample size:

It has been published that the prevalence of DAO deficiency of genetic origin in the general population is close to 15% (International Society of DAO Deficiency. What is DAO deficiency? Available online: https://www.deficitdao .org/el-deficit-dedao/que-es/ (accessed on 23rd January 2024). The hypothesis of the present study is that the prevalence of DAO deficiency of genetic origin could be higher than that of the general population in patients with insomnia symptoms, as occurs in patients with other pathologies. (Izquierdo-Casas et al., 2018; Okutan et al. 2022 and 2023).

As a working hypothesis, it is estimated that the prevalence of DAO deficiency of genetic origin in patients who report symptoms of insomnia is equal to or greater than 60%. A sample size of n = 126 volunteers is defined, which would allow establishing the prevalence of DAO of genetic origin in patients who report symptoms of insomnia, with a precision of approximately +/- 7%.

### Evaluation of study variables

Main variable: Number (absolute and percentage) of patients reporting symptoms of insomnia and DAO deficiency.

DAO deficiency was defined as the presence of at least one of the AOC1 gene SNPs described previously, with reference rs10156191, rs1049742, rs1049793 and rs2052129 (Garcia-Martin et al., 2007; Maintz et al., 2011).

### Schedule and expected completion date of the study

After the approval of the project by the Autonomous Research Ethics Committee (CElm) of the General Hospital of Catalonia, there was a recruitment period of 4 months, and after the completion of the study in 2 months, the statistical analysis and publication of results was carried out..
- Recruitment start: After approval by the CEIm.
- Recruitment period: 4 months.
- Follow-up period for each patient: 1 day.Statistical analysis period: 2 months.

### Results:

Of the total number of volunteers with insomnia, the prevalence of DAO deficiency is 83.3% (Table 1):

**Table 1. Characteristics of the DAO Deficit**

| | **Total (N=126)** |
|---|---|
| **DAO deficit** | |
| N | 126 |
| No | 21 (16.7%) |
| Yeah | 105 (83.3%) |
| 95% CI - Clopper-Pearson (accurate) | (75.7%; 89.4%) |

| **Mutations** | |
|---|---|
| N | 126 |
| No mutations | 21 (16.7%) |
| Heterozygous mutation | 93 (73.8%) |
| Homozygous Mutation | 12 (9.5%) |

| **c.-691G>T (rs2052129)** | |
|---|---|
| N | 126 |
| GG | 41 (32.5%) |
| G.T. | 80 (63.5%) |
| T.T. | 5 (4.0%) |

| **c.-691G>T (rs2052129)** | |
|---|---|
| N | 126 |
| GG | 41 (32.5%) |
| G>T | 85 (67.5%) |

| **c.47C>T (rs10156191)** | |
|---|---|
| N | 126 |
| DC | 46 (36.5%) |
| C.T. | 73 (57.9%) |
| T.T. | 7 (5.6%) |

| **c.47C>T (rs10156191)** | |
|---|---|
| N | 126 |
| DC | 46 (36.5%) |
| C>T | 80 (63.5%) |

| **c.995C>T (rs1049742)** | |
|---|---|
| N | 126 |
| DC | 53 (42.1%) |
| C.T. | 72 (57.1%) |
| T.T. | 1 (0.8%) |

| **c.995C>Tc.995C>T (rs1049742)** | |
|---|---|
| N | 126 |
| DC | 53 (42.1%) |
| C>T | 73 (57.9%) |

| **c.1990C>Gc.1990C>G (rs1049793)** | |
|---|---|
| N | 126 |
| DC | 70 (55.6%) |
| CG | 52 (41.3%) |
| GG | 4 (3.2%) |

| | **Total (N=126)** |
|---|---|
| **c.1990C>Gc.1990C>G (rs1049793)** | |
| N | 126 |
| DC | 70 (55.6%) |
| C>G | 56 (44.4%) |

Having DAO deficiency is associated with early awakening, particularly in people who have all 4 mutations (DAO score ≥ 4, Tables 2 and 3) or who have the c.1990C>G (rs1049793) mutation alone or in combination with others:

**Table 1 Characteristics of Insomnia According to DAO-Score**

| | **0 (N=21)** | **1 (N=16)** | **2 (N=20)** | **3 (N=27)** | **≥4 (N=42)** | **p-value** |
|---|---|---|---|---|---|---|
| **Start** | | | | | | |
| N | twenty-one | 16 | twenty | 27 | 42 | |
| No | 8 (38.1%) | 8 (50.0%) | 5 (25.0%) | 11 (40.7%) | 15 (35.7%) | 0.7287 ⁽²⁾ |
| Yeah | 13 (61.9%) | 8 (50.0%) | 15 (75.0%) | 16 (59.3%) | 27 (64.3%) | |
| 0 | - | 0.5195 ⁽¹⁾ | 0.5055 ⁽¹⁾ | 1,0000 ⁽¹⁾ | 1,0000 ⁽¹⁾ | |
| 1 | 0.5195 ⁽¹⁾ | - | 0.1691 ⁽¹⁾ | 0.7516 ⁽¹⁾ | 0.3762 ⁽¹⁾ | |
| 2 | 0.5055 ⁽¹⁾ | 0.1691 ⁽¹⁾ | - | 0.3547 ⁽¹⁾ | 0.5626 ⁽¹⁾ | |
| 3 | 1,0000 ⁽¹⁾ | 0.7516 ⁽¹⁾ | 0.3547 ⁽¹⁾ | | 0.8000 ⁽¹⁾ | |
| ≥ 4 | 1,0000 ⁽¹⁾ | 0.3762 ⁽¹⁾ | 0.5626 ⁽¹⁾ | 0.8000 ⁽¹⁾ | - | |

| **Maintenance** | | | | | | |
|---|---|---|---|---|---|---|
| N | twenty-one | 16 | twenty | 27 | 42 | |
| No | 1 (4.8%) | 2 (12.5%) | 5 (25.0%) | 5 (18.5%) | 3 (7.1%) | 0.9609 ⁽²⁾ |
| Yeah | 20 (95.2%) | 14 (87.5%) | 15 (75.0%) | 22 (81.5%) | 39 (92.9%) | |
| 0 | - | 0.5676 ⁽¹⁾ | 0.0931 ⁽¹⁾ | 0.2115 ⁽¹⁾ | 1,0000 ⁽¹⁾ | |
| 1 | 0.5676 ⁽¹⁾ | - | 0.4264 ⁽¹⁾ | 0.6950 ⁽¹⁾ | 0.6092 ⁽¹⁾ | |
| 2 | 0.0931 ⁽¹⁾ | 0.4264 ⁽¹⁾ | - | 0.7230 ⁽¹⁾ | 0.0984 ⁽¹⁾ | |
| 3 | 0.2115 ⁽¹⁾ | 0.6950 ⁽¹⁾ | 0.7230 ⁽¹⁾ | | 0.2473 ⁽¹⁾ | |
| ≥ 4 | 1,0000 ⁽¹⁾ | 0.6092 ⁽¹⁾ | 0.0984 ⁽¹⁾ | 0.2473 ⁽¹⁾ | - | |

| | **0 (N=21)** | **1 (N=16)** | **2 (N=20)** | **3 (N=27)** | **≥4 (N=42)** | **p-value** |
|---|---|---|---|---|---|---|
| **Wake up early** | | | | | | |
| N | twenty-one | 16 | twenty | 27 | 42 | |
| No | 12 (57.1%) | 10 (62.5%) | 12 (60.0%) | 15 (55.6%) | 13 (31.0%) | **0.0208 ⁽²⁾** |
| Yeah | 9 (42.9%) | 6 (37.5%) | 8 (40.0%) | 12 (44.4%) | 29 (69.0%) | |
| 0 | - | 1,0000 ⁽¹⁾ | 1,0000 ⁽¹⁾ | 1,0000 ⁽¹⁾ | 0.0587 ⁽¹⁾ | |
| 1 | 1,0000 ⁽¹⁾ | - | 1,0000 ⁽¹⁾ | 0.7548 ⁽¹⁾ | 0.0380 ⁽¹⁾ | |
| 2 | 1,0000 ⁽¹⁾ | 1,0000 ⁽¹⁾ | - | 1,0000 ⁽¹⁾ | 0.0512 ⁽¹⁾ | |
| 3 | 1,0000 ⁽¹⁾ | 0.7548 ⁽¹⁾ | 1,0000 ⁽¹⁾ | - | 0.0493 ⁽¹⁾ | |
| ≥ 4 | 0.0587 ⁽¹⁾ | 0.0380 ⁽¹⁾ | 0.0512 ⁽¹⁾ | 0.0493 ⁽¹⁾ | - | |

| **Number of nights with insomnia** | | | | | | |
|---|---|---|---|---|---|---|
| N | twenty-one | 16 | twenty | 27 | 41 | |
| Less than 3 days | 0 (0.0%) | 3 (18.8%) | 1 (5.0%) | 4 (14.8%) | 7 (17.1%) | 0.8329 ⁽³⁾ |
| per week | | | | | | |
| 3 or more days per | 10 | 5 (31.2%) | 9 (45.0%) | 11 | 11 | |
| week | (47.6%) | | | (40.7%) | (26.8%) | |
| Every day | 11 (52.4%) | 8 (50.0%) | 10 (50.0%) | 12 (44.4%) | 23 (56.1%) | |
| 0 | - | 0.1029 ⁽²⁾ | 0.5838 ⁽²⁾ | 0.1832 ⁽²⁾ | 0.0675 ⁽²⁾ | |
| 1 | 0.1029 ⁽²⁾ | - | 0.3782 ⁽²⁾ | 0.8165 ⁽²⁾ | 0.9152 ⁽²⁾ | |
| 2 | 0.5838 ⁽²⁾ | 0.3782 ⁽²⁾ | - | 0.5585 ⁽²⁾ | 0.2299 ⁽²⁾ | |
| 3 | 0.1832 ⁽²⁾ | 0.8165 ⁽²⁾ | 0.5585 ⁽²⁾ | - | 0.4832 ⁽²⁾ | |
| ≥ 4 | 0.0675 ⁽²⁾ | 0.9152 ⁽²⁾ | 0.2299 ⁽²⁾ | 0.4832 ⁽²⁾ | - | |
| (1) Fisher's exact test; (2) Pearson chi-squared test; (3) Spearman's rank correlation rho | | | | | | |

**Table 2Characteristics of Insomnia According to Number of Mutations**

| | **0 (N=21)** | **1 (N=17)** | **2 (N=23)** | **3 (N=29)** | **4 (N=36)** | **p-value** |
|---|---|---|---|---|---|---|
| **Start** | | | | | | |
| N | twenty-one | 17 | 23 | 29 | 36 | |
| No | 8 (38.1%) | 8 (47.1%) | 6 (26.1%) | 11 (37.9%) | 14 (38.9%) | 0.9319 ⁽²⁾ |
| Yeah | 13 (61.9%) | 9 (52.9%) | 17 (73.9%) | 18 (62.1%) | 22 (61.1%) | |
| 0 | - | 0.7430 ⁽¹⁾ | 0.5206 ⁽¹⁾ | 1,0000 ⁽¹⁾ | 1,0000 ⁽¹⁾ | |
| 1 | 0.7430 ⁽¹⁾ | - | 0.1978 ⁽¹⁾ | 0.7571 ⁽¹⁾ | 0.7659 ⁽¹⁾ | |
| 2 | 0.5206 ⁽¹⁾ | 0.1978 ⁽¹⁾ | - | 0.3927 ⁽¹⁾ | 0.4020 ⁽¹⁾ | |
| 3 | 1,0000 ⁽¹⁾ | 0.7571 ⁽¹⁾ | 0.3927 ⁽¹⁾ | - | 1,0000 ⁽¹⁾ | |
| 4 | 1,0000 ⁽¹⁾ | 0.7659 ⁽¹⁾ | 0.4020 ⁽¹⁾ | 1,0000 ⁽¹⁾ | - | |

| **Maintenance** | | | | | | |
|---|---|---|---|---|---|---|
| N | twenty-one | 17 | 23 | 29 | 36 | |
| No | 1 (4.8%) | 3 (17.6%) | 5 (21.7%) | 5 (17.2%) | 2 (5.6%) | 0.8039 ⁽²⁾ |
| Yeah | 20 (95.2%) | 14 (82.4%) | 18 (78.3%) | 24 (82.8%) | 34 (94.4%) | |
| 0 | - | 0.3068 ⁽¹⁾ | 0.1884 ⁽¹⁾ | 0.3803 ⁽¹⁾ | 1,0000 ⁽¹⁾ | |
| 1 | 0.3068 ⁽¹⁾ | | 1,0000 ⁽¹⁾ | 1,0000 ⁽¹⁾ | 0.3127 ⁽¹⁾ | |
| 2 | 0.1884 ⁽¹⁾ | 1,0000 ⁽¹⁾ | - | 0.7341 ⁽¹⁾ | 0.0980 ⁽¹⁾ | |
| 3 | 0.3803 ⁽¹⁾ | 1,0000 ⁽¹⁾ | 0.7341 ⁽¹⁾ | - | 0.2274 ⁽¹⁾ | |
| 4 | 1,0000 ⁽¹⁾ | 0.3127 ⁽¹⁾ | 0.0980 ⁽¹⁾ | 0.2274 ⁽¹⁾ | - | |

| **Wake up early** | | | | | | |
|---|---|---|---|---|---|---|
| N | twenty-one | 17 | 23 | 29 | 36 | |
| No | 12 (57.1%) | 10 (58.8%) | 15 (65.2%) | 14 (48.3%) | 11 (30.6%) | **0.0206 ⁽²⁾** |
| Yeah | 9 (42.9%) | 7 (41.2%) | 8 (34.8%) | 15 (51.7%) | 25 (69.4%) | |
| 0 | - | 1,0000 ⁽¹⁾ | 0.7577 ⁽¹⁾ | 0.5780 ⁽¹⁾ | 0.0572 ⁽¹⁾ | |
| 1 | 1,0000 ⁽¹⁾ | | 0.7486 ⁽¹⁾ | 0.5520 ⁽¹⁾ | 0.0722 ⁽¹⁾ | |
| 2 | 0.7577 ⁽¹⁾ | 0.7486 ⁽¹⁾ | - | 0.2689 ⁽¹⁾ | 0.0150 ⁽¹⁾ | |
| 3 | 0.5780 ⁽¹⁾ | 0.5520 ⁽¹⁾ | 0.2689 ⁽¹⁾ | - | 0.2007 ⁽¹⁾ | |
| 4 | 0.0572 ⁽¹⁾ | 0.0722 ⁽¹⁾ | 0.0150 ⁽¹⁾ | 0.2007 ⁽¹⁾ | - | |

| **Number of nights with insomnia** | | | | | | |
|---|---|---|---|---|---|---|
| N | twenty-one | 17 | 23 | 29 | 35 | |
| Less than 3 days | 0 (0.0%) | 3 (17.6%) | 2 (8.7%) | 4 (13.8%) | 6 (17.1%) | 0.7654 ⁽³⁾ |
| per week | | | | | | |
| 3 or more days per | 10 | 6 (35.3%) | 10 | 9 (31.0%) | 11 | |
| week | (47.6%) | | (43.5%) | | (31.4%) | |
| Every day | 11 (52.4%) | 8 (47.1%) | 11 (47.8%) | 16 (55.2%) | 18 (51.4%) | |
| 0 | - | 0.1289 ⁽²⁾ | 0.3842 ⁽²⁾ | 0.1499 ⁽²⁾ | 0.1044 ⁽²⁾ | |
| 1 | 0.1289 ⁽²⁾ | - | 0.6732 ⁽²⁾ | 0.8609 ⁽²⁾ | 0.9523 ⁽²⁾ | |
| 2 | 0.3842 ⁽²⁾ | 0.6732 ⁽²⁾ | - | 0.6170 ⁽²⁾ | 0.5193 ⁽²⁾ | |
| 3 | 0.1499 ⁽²⁾ | 0.8609 ⁽²⁾ | 0.6170 ⁽²⁾ | - | 0.9247 ⁽²⁾ | |
| 4 | 0.1044 ⁽²⁾ | 0.9523 ⁽²⁾ | 0.5193 ⁽²⁾ | 0.9247 ⁽²⁾ | - | |

| | | | | | | |
|---|---|---|---|---|---|---|
| (1) Fisher's exact test; (2) Pearson chi-squared test; (3) Spearman's rank correlation rho | | | | | | |

Furthermore, the c.1990C>G (rs1049793) mutation alone or in combination with others is associated with maintenance insomnia (Table 4):

**Table 3Insomnia of maintenance according to AOC1 gene SNPs:**

| | | **No (N=16)** | **Yes (N=110)** | **P-value** |
|---|---|---|---|---|
| | | **DAO deficit** | | |
| N | | 16 | 110 | |
| No | | 1 (6.2%) | 20 (18.2%) | 0.4700 ⁽¹⁾ |
| Yeah | | 15 (93.8%) | 90 (81.8%) | |

| | | **Mutations** | | |
|---|---|---|---|---|
| N | | 16 | 110 | |
| Heterozygous mutation | | 13 (81.2%) | 80 (72.7%) | 0.4721 ⁽²⁾ |
| Homozygous Mutation | | 2 (12.5%) | 10 (9.1%) | |
| No mutations | | 1 (6.2%) | 20 (18.2%) | |

| | | **c.-691G>T (rs2052129)** | | |
|---|---|---|---|---|
| N | | 16 | 110 | |
| GG | | 3 (18.8%) | 38 (34.5%) | 0.4288 ⁽²⁾ |
| G.T. | | 12 (75.0%) | 68 (61.8%) | |
| T.T. | | 1 (6.2%) | 4 (3.6%) | |

| | | **c.-691G>T (rs2052129)** | | |
|---|---|---|---|---|
| N | | 16 | 110 | |
| GG | | 3 (18.8%) | 38 (34.5%) | 0.2626 ⁽¹⁾ |
| G>T | | 13 (81.2%) | 72 (65.5%) | |

| | | **c.47C>T (rs10156191)** | | |
|---|---|---|---|---|
| N | | 16 | 110 | |
| DC | | 5 (31.2%) | 41 (37.3%) | 0.4192 ⁽²⁾ |
| C.T. | | 9 (56.2%) | 64 (58.2%) | |
| T.T. | | 2 (12.5%) | 5 (4.5%) | |

| | | **c.47C>T (rs10156191)** | | |
|---|---|---|---|---|
| N | | 16 | 110 | |
| DC | | 5 (31.2%) | 41 (37.3%) | 0.7840 ⁽¹⁾ |
| C>T | | 11 (68.8%) | 69 (62.7%) | |

| | | **c.995C>T (rs1049742)** | | |
|---|---|---|---|---|
| N | | 16 | 110 | |
| DC | | 7 (43.8%) | 46 (41.8%) | 0.9229 ⁽²⁾ |
| C.T. | | 9 (56.2%) | 63 (57.3%) | |
| T.T. | | 0 (0.0%) | 1 (0.9%) | |

| | | **c.995C>T (rs1049742)** | | |
|---|---|---|---|---|
| N | | 16 | 110 | |
| DC | | 7 (43.8%) | 46 (41.8%) | 1,0000 ⁽¹⁾ |
| C>T | | 9 (56.2%) | 64 (58.2%) | |

| | | **No (N=16)** | **Yes (N=110)** | **P-value** |
|---|---|---|---|---|
| | | **c.1990C>G (rs1049793)** | | |
| N | | 16 | 110 | |
| DC | | 13 (81.2%) | 57 (51.8%) | 0.0816 ⁽²⁾ |
| CG | | 3 (18.8%) | 49 (44.5%) | |
| GG | | 0 (0.0%) | 4 (3.6%) | |

| | | **c.1990C>G (rs1049793)** | | |
|---|---|---|---|---|
| N | | 16 | 110 | |
| DC | | 13 (81.2%) | 57 (51.8%) | **0.0320 ⁽¹⁾** |
| C>G | | 3 (18.8%) | 53 (48.2%) | |

| | | **DAO-Score** | | |
|---|---|---|---|---|
| N | | 16 | 110 | |
| 0 | | 1 (6.2%) | 20 (18.2%) | 0.2053 ⁽²⁾ |
| 1 | | 2 (12.5%) | 14 (12.7%) | |
| 2 | | 5 (31.2%) | 15 (13.6%) | |
| 3 | | 5 (31.2%) | 22 (20.0%) | |
| 4 | | 3 (18.8%) | 39 (35.5%) | |

| | | **Number of altered variants** | | |
|---|---|---|---|---|
| N | | 16 | 110 | |
| 0 | | 1 (6.2%) | 20 (18.2%) | 0.2432 ⁽²⁾ |
| 1 | | 3 (18.8%) | 14 (12.7%) | |
| 2 | | 5 (31.2%) | 18 (16.4%) | |
| 3 | | 5 (31.2%) | 24 (21.8%) | |
| 4 | | 2 (12.5%) | 34 (30.9%) | |

| | | **(c.-691G>T) or (c.47C>T)** | | |
|---|---|---|---|---|
| N | | 16 | 110 | |
| No | | 2 (12.5%) | 32 (29.1%) | 0.2317 ⁽¹⁾ |
| Yeah | | 14 (87.5%) | 78 (70.9%) | |

| | | **(c.-691G>T) or (c-99C>T)** | | |
|---|---|---|---|---|
| N | | 16 | 110 | |
| No | | 3 (18.8%) | 25 (22.7%) | 1,0000 ⁽¹⁾ |
| Yeah | | 13 (81.2%) | 85 (77.3%) | |

| | | **(c.-691G>T) or (c.1990C>G)** | | |
|---|---|---|---|---|
| N | | 16 | 110 | |
| No | | 2 (12.5%) | 28 (25.5%) | 0.3546 ⁽¹⁾ |
| Yeah | | 14 (87.5%) | 82 (74.5%) | |

| | | **(c.47CC>T) or (c.995C>T)** | | |
|---|---|---|---|---|
| N | | 16 | 110 | |
| No | | 3 (18.8%) | 29 (26.4%) | 0.7594 ⁽¹⁾ |
| Yeah | | 13 (81.2%) | 81 (73.6%) | |

| | | **No (N = 16)** | **Yes (N=110)** | **P-value** |
|---|---|---|---|---|
| | | **(c.47CC>T) or (c.1990C>G)** | | |
| N | | 16 | 110 | |
| No | | 4 (25.0%) | 33 (30.0%) | 0.7770 ⁽¹⁾ |
| Yeah | | 12 (75.0%) | 77 (70.0%) | |

| | | **(c.995C>T) or (c.1990C>G)** | | |
|---|---|---|---|---|
| N | | 16 | 110 | |
| No | | 6 (37.5%) | 33 (30.0%) | 0.5698 ⁽¹⁾ |
| Yeah | | 10 (62.5%) | 77 (70.0%) | |

| | | **(c.-691G>T) or (c.47C>T) or (c.995C>T)** | | |
|---|---|---|---|---|
| | N | 16 | 110 | |
| No | | 2 (12.5%) | 24 (21.8%) | 0.5210 ⁽¹⁾ |
| Yeah | | 14 (87.5%) | 86 (78.2%) | |

| | | **(c.-691G>T) or (c.47C>T) or (c.1990C>G)** | | |
|---|---|---|---|---|
| | N | 16 | 110 | |
| No | | 1 (6.2%) | 26 (23.6%) | 0.1899 ⁽¹⁾ |
| Yeah | | 15 (93.8%) | 84 (76.4%) | |

| | | **(c.-691G>T) or (c.995C>T) or (c.1990C>G)** | | |
|---|---|---|---|---|
| | N | 16 | 110 | |
| No | | 2 (12.5%) | 20 (18.2%) | 0.7361 ⁽¹⁾ |
| Yeah | | 14 (87.5%) | 90 (81.8%) | |

| | | **(c.47C>T) or (c.995C>T) or (c.1990C>G)** | | |
|---|---|---|---|---|
| | N | 16 | 110 | |
| No | | 2 (12.5%) | 24 (21.8%) | 0.5210 ⁽¹⁾ |
| Yeah | | 14 (87.5%) | 86 (78.2%) | |

| | | **(c.-691G>T) and (c.47C>T)** | | |
|---|---|---|---|---|
| N | | 16 | 110 | |
| No | | 6 (37.5%) | 47 (42.7%) | 0.7904 ⁽¹⁾ |
| Yeah | | 10 (62.5%) | 63 (57.3%) | |

| | | **(c.-691G>T) and (c.995C>T)** | | |
|---|---|---|---|---|
| N | | 16 | 110 | |
| No | | 7 (43.8%) | 59 (53.6%) | 0.5939 ⁽¹⁾ |
| Yeah | | 9 (56.2%) | 51 (46.4%) | |

| | | **(c.-691G>T) and (c.1990C>G)** | | |
|---|---|---|---|---|
| N | | 16 | 110 | |
| No | | 14 (87.5%) | 67 (60.9%) | **0.0498 ⁽¹⁾** |
| Yeah | | 2 (12.5%) | 43 (39.1%) | |

| | | **(c.47CC>T) and (c.995C>T)** | | |
|---|---|---|---|---|
| | N | 16 | 110 | |
| No | | 9 (56.2%) | 58 (52.7%) | 1,0000 ⁽¹⁾ |
| Yeah | | 7 (43.8%) | 52 (47.3%) | |

| | | **No (N = 16)** | **Yes (N=110)** | **P-value** |
|---|---|---|---|---|
| | | **(c.47CC>T) and (c.1990C>G)** | | |
| | N | 16 | 110 | |
| No | | 14 (87.5%) | 65 (59.1%) | **0.0295 ⁽¹⁾** |
| Yeah | | 2 (12.5%) | 45 (40.9%) | |

| | | **(c.995C>T) and (c.1990C>G)** | | |
|---|---|---|---|---|
| N | | 16 | 110 | |
| No | | 14 (87.5%) | 70 (63.6%) | 0.0867 ⁽¹⁾ |
| Yeah | | 2 (12.5%) | 40 (36.4%) | |

| | | **(c.-691G>T) and (c.47C>T) and (c.995C>T)** | | |
|---|---|---|---|---|
| | N | 16 | 110 | |
| No | | 9 (56.2%) | 63 (57.3%) | 1,0000 ⁽¹⁾ |
| Yeah | | 7 (43.8%) | 47 (42.7%) | |

| | | **(c.-691G>T) and (c.47C>T) and (c.1990C>G)** | | |
|---|---|---|---|---|
| | N | 16 | 110 | |
| No | | 14 (87.5%) | 69 (62.7%) | 0.0872 ⁽¹⁾ |
| Yeah | | 2 (12.5%) | 41 (37.3%) | |

| | | **(c.-691G>T) and (c.995C>T) and (c.1990C>G)** | | |
|---|---|---|---|---|
| | N | 16 | 110 | |
| No | | 14 (87.5%) | 75 (68.2%) | 0.1471 ⁽¹⁾ |
| Yeah | | 2 (12.5%) | 35 (31.8%) | |

| | | **(c.47C>T) and (c.995C>T) and (c.1990C>G)** | | |
|---|---|---|---|---|
| | N | 16 | 110 | |
| No | | 14 (87.5%) | 73 (66.4%) | 0.1454 ⁽¹⁾ |
| Yeah | | 2 (12.5%) | 37 (33.6%) | |

| | | **(c.-691G>T) and (c.47C>T) and (c.995C>T) and (c.1990C>G)** | | |
|---|---|---|---|---|
| | N | 16 | 110 | |
| No | | 14 (87.5%) | 76 (69.1%) | 0.1512 ⁽¹⁾ |
| Yeah | | 2 (12.5%) | 34 (30.9%) | |

Finally, having DAO deficiency seems to protect against cognitive deficit as a daytime manifestation of insomnia, specifically in patients with 4 mutations, and DAO deficiency shows a trend to being associated with mood changes in people with insomnia (Table 5):

**Table 5. Daily repercussions of insomnia as per DAO-Score**

| | | | **0 (N=21)** | **1 (N=16)** | **2 (N=20)** | **3 (N=27)** | **≥4 (N=42)** | **p-value** |
|---|---|---|---|---|---|---|---|---|
| **Daily repercussion** | | | | | | | | |
| | N | | 21 | 16 | 20 | 27 | 42 | |
| | No | | 0 (0,0%) | 0 (0,0%) | 0 (0,0%) | 0 (0,0%) | 1 (2,4%) | 0,2811⁽¹⁾ |
| | Yes | | 21 (100,0%) | 16 (100,0%) | 20 (100,0%) | 27 (100,0%) | 41 (97,6%) | |
| | | 0 | - | 1,0000⁽¹⁾ | 1,0000⁽¹⁾ | 1,0000⁽¹⁾ | 1,0000⁽¹⁾ | |
| | | 1 | 1,0000⁽¹⁾ | - | 1,0000⁽¹⁾ | 1,0000⁽¹⁾ | 1,0000⁽¹⁾ | |
| | | 2 | 1,0000⁽¹⁾ | 1,0000⁽¹⁾ | - | 1,0000⁽¹⁾ | 1,0000⁽¹⁾ | |
| | | 3 | 1,0000⁽¹⁾ | 1,0000⁽¹⁾ | 1,0000⁽¹⁾ | - | 1,0000⁽¹⁾ | |
| | | ≥4 | 1,0000⁽¹⁾ | 1,0000⁽¹⁾ | 1,0000⁽¹⁾ | 1,0000⁽¹⁾ | - | |

| **Somnolence** | | | | | | | | |
|---|---|---|---|---|---|---|---|---|
| | N | | 21 | 16 | 20 | 27 | 42 | |
| | No | | 13 (61,9%) | 9 (56,2%) | 7 (35,0%) | 15 (55,6%) | 21 (50,0%) | 0,5175⁽²⁾ |
| | Yes | | 8 (38,1%) | 7 (43,8%) | 13 (65,0%) | 12 (44,4%) | 21 (50,0%) | |
| | | 0 | - | 0,7486⁽¹⁾ | 0,1215⁽¹⁾ | 0,7710⁽¹⁾ | 0,4294⁽¹⁾ | |
| | | 1 | 0,7486⁽¹⁾ | - | 0,3128⁽¹⁾ | 1,0000⁽¹⁾ | 0,7725⁽¹⁾ | |
| | | 2 | 0,1215⁽¹⁾ | 0,3128⁽¹⁾ | - | 0,2384⁽¹⁾ | 0,2910⁽¹⁾ | |
| | | 3 | 0,7710⁽¹⁾ | 1,0000⁽¹⁾ | 0,2384⁽¹⁾ | - | 0,8055⁽¹⁾ | |
| | | ≥4 | 0,4294⁽¹⁾ | 0,7725⁽¹⁾ | 0,2910⁽¹⁾ | 0,8055⁽¹⁾ | - | |

| **Fatigue** | | | | | | | | |
|---|---|---|---|---|---|---|---|---|
| | N | | 21 | 16 | 20 | 27 | 42 | |
| | No | | 4 (19,0%) | 3 (18,8%) | 4 (20,0%) | 7 (25,9%) | 6 (14,3%) | 0,7466⁽²⁾ |
| | Yes | | 17 (81,0%) | 13 (81,2%) | 16 (80,0%) | 20 (74,1%) | 36 (85,7%) | |
| | | 0 | | 1,0000⁽¹⁾ | 1,0000⁽¹⁾ | 0,7334⁽¹⁾ | 0,7193⁽¹⁾ | |
| | | 1 | 1,0000⁽¹⁾ | - | 1,0000⁽¹⁾ | 0,7190⁽¹⁾ | 0,6960⁽¹⁾ | |
| | | 2 | 1,0000⁽¹⁾ | 1,0000⁽¹⁾ | - | 0,7365⁽¹⁾ | 0,7140⁽¹⁾ | |
| | | 3 | 0,7334⁽¹⁾ | 0,7190⁽¹⁾ | 0,7365⁽¹⁾ | - | 0,3444⁽¹⁾ | |
| | | ≥4 | 0,7193⁽¹⁾ | 0,6960⁽¹⁾ | 0,7140⁽¹⁾ | 0,3444⁽¹⁾ | - | |

| **Cognitive deficit** | | | | | | | | |
|---|---|---|---|---|---|---|---|---|
| | N | | 21 | 16 | 20 | 27 | 42 | |
| | No | | 15 (71,4%) | 14 (87,5%) | 18 (90,0%) | 22 (81,5%) | 40 (95,2%) | **0,0313⁽²⁾** |
| | Yes | | 6 (28,6%) | 2 (12,5%) | 2 (10,0%) | 5 (18,5%) | 2 (4,8%) | |
| | | 0 | - | 0,4227⁽¹⁾ | 0,2379⁽¹⁾ | 0,4982⁽¹⁾ | **0,0134⁽¹⁾** | |
| | | 1 | 0,4227⁽¹⁾ | - | 1,0000⁽¹⁾ | 0,6950⁽¹⁾ | 0,3033⁽¹⁾ | |
| | | 2 | 0,2379⁽¹⁾ | 1,0000⁽¹⁾ | - | 0,6819⁽¹⁾ | 0,5884⁽¹⁾ | |
| | | 3 | 0,4982⁽¹⁾ | 0,6950⁽¹⁾ | 0,6819⁽¹⁾ | - | 0,1018⁽¹⁾ | |
| | | ≥4 | **0,0134⁽¹⁾** | 0,3033⁽¹⁾ | 0,5884⁽¹⁾ | 0,1018⁽¹⁾ | - | |

| | | | **0 (N=21)** | **1 (N=16)** | **2 (N=20)** | **3 (N=27)** | **≥4 (N=42)** | **p-value** |
|---|---|---|---|---|---|---|---|---|
| **Lack of concentration** | | | | | | | | |
| | N | | 21 | 16 | 20 | 27 | 42 | |
| | No | | 6 (28,6%) | 4 (25,0%) | 6 (30,0%) | 11 (40,7%) | 13 (31,0%) | 0,5871⁽²⁾ |
| | Yes | | 15 (71,4%) | 12 (75,0%) | 14 (70,0%) | 16 (59,3%) | 29 (69,0%) | |
| | | 0 | - | 1,0000⁽¹⁾ | 1,0000⁽¹⁾ | 0,5442⁽¹⁾ | 1,0000⁽¹⁾ | |
| | | 1 | 1,0000⁽¹⁾ | | 1,0000⁽¹⁾ | 0,3415⁽¹⁾ | 0,7555⁽¹⁾ | |
| | | 2 | 1,0000⁽¹⁾ | 1,0000⁽¹⁾ | - | 0,5460⁽¹⁾ | 1,0000⁽¹⁾ | |
| | | 3 | 0,5442⁽¹⁾ | 0,3415⁽¹⁾ | 0,5460⁽¹⁾ | - | 0,4457⁽¹⁾ | |
| | | >_4 | 1,0000⁽¹⁾ | 0,7555⁽¹⁾ | 1,0000⁽¹⁾ | 0,4457⁽¹⁾ | - | |

| **Attention or memory deficit** | | | | | | | | |
|---|---|---|---|---|---|---|---|---|
| | N | | 21 | 16 | 20 | 27 | 42 | |
| | No | | 12 (57,1%) | 4 (25,0%) | 11 (55,0%) | 16 (59,3%) | 16 (38,1%) | 0,5588⁽²⁾ |
| | Yes | | 9 (42,9%) | 12 (75,0%) | 9 (45,0%) | 11 (40,7%) | 26 (61,9%) | |
| | | 0 | - | 0,0931⁽¹⁾ | 1,0000⁽¹⁾ | 1,0000⁽¹⁾ | 0,1848⁽¹⁾ | |
| | | 1 | 0,0931⁽¹⁾ | - | 0,0958(1) | 0,0563⁽¹⁾ | 0,5375⁽¹⁾ | |
| | | 2 | 1,0000⁽¹⁾ | 0,0958⁽¹⁾ | - | 1,0000⁽¹⁾ | 0,2758⁽¹⁾ | |
| | | 3 | 1,0000⁽¹⁾ | 0,0563⁽¹⁾ | 1,0000⁽¹⁾ | - | 0,1372⁽¹⁾ | |
| | | >_4 | 0,1848⁽¹⁾ | 0,5375⁽¹⁾ | 0,2758⁽¹⁾ | 0,1372⁽¹⁾ | - | |

| **Mood changes** | | | | | | | | |
|---|---|---|---|---|---|---|---|---|
| | N | | 21 | 16 | 20 | 27 | 42 | |
| | No | | 3 (14,3%) | 5 (31,2%) | 1 (5,0%) | 7 (25,9%) | 15 (35,7%) | **0,0684⁽²⁾** |
| | Yes | | 18 (85,7%) | 11 (68,8%) | 19 (95,0%) | 20 (74,1%) | 27 (64,3%) | |
| | | 0 | - | 0,2540⁽¹⁾ | 0,6060⁽¹⁾ | 0,4780⁽¹⁾ | 0,1375⁽¹⁾ | |
| | | 1 | 0,2540⁽¹⁾ | - | 0,0689⁽¹⁾ | 0,7366⁽¹⁾ | 1,0000⁽¹⁾ | |
| | | 2 | 0,6060⁽¹⁾ | 0,0689⁽¹⁾ | - | 0,1139⁽¹⁾ | 0,0120⁽¹⁾ | |
| | | 3 | 0,4780⁽¹⁾ | 0,7366⁽¹⁾ | 0,1139⁽¹⁾ | - | 0,4390⁽¹⁾ | |
| | | >_4 | 0,1375⁽¹⁾ | 1,0000⁽¹⁾ | 0,0120⁽¹⁾ | 0,4390⁽¹⁾ | - | |

| **Headache** | | | | | | | | |
|---|---|---|---|---|---|---|---|---|
| | N | | 21 | 16 | 20 | 27 | 42 | |
| | No | | 10 (47,6%) | 11 (68,8%) | 11 (55,0%) | 9 (33,3%) | 26 (61,9%) | 0,8254⁽²⁾ |
| | Yes | | 11 (52,4%) | 5 (31,2%) | 9 (45,0%) | 18 (66,7%) | 16 (38,1%) | |
| | | 0 | - | 0,3161⁽¹⁾ | 0,7579⁽¹⁾ | 0,3802⁽¹⁾ | 0,2963⁽¹⁾ | |
| | | 1 | 0,3161⁽¹⁾ | - | 0,5007⁽¹⁾ | 0,0316⁽¹⁾ | 0,7636⁽¹⁾ | |
| | | 2 | 0,7579⁽¹⁾ | 0,5007⁽¹⁾ | - | 0,2324⁽¹⁾ | 0,7824⁽¹⁾ | |
| | | 3 | 0,3802⁽¹⁾ | 0,0316⁽¹⁾ | 0,2324⁽¹⁾ | - | 0,0273⁽¹⁾ | |
| | | >_4 | 0,2963⁽¹⁾ | 0,7636⁽¹⁾ | 0,7824⁽¹⁾ | 0,0273⁽¹⁾ | - | |

| | | | | | | | | |
|---|---|---|---|---|---|---|---|---|
| (1) Fisher's exact test; (2) Pearson chi-squared test | | | | | | | | |

### EXAMPLE 2 DAO supplementation in patients with insomnia

Supplementation with DAOFood^{®} was given to 4 patients with a diagnosis of chronic insomnia for a total of 20 days, and their sleep was evaluated prior to the start of supplementation and after 20 days of supplementation. The established guidelines were to take one tablet 20 minutes before each of the 3 main meals (breakfast, lunch, dinner).

Below is a table with the results of the 4 patients who completed the 20-day supplementation:

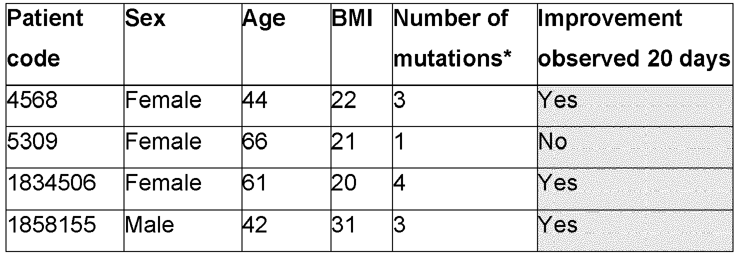

| **Patient code** | ***Descriptive of the variants** |
|---|---|
| 4568 | c.-691G>T, c.47C>T, c.995C>T |
| 5309 | c.-691G>T |
| 1834506 | c.-691G>T, c.47C>T, c.995C>T, c.1990C>G |
| 1858155 | c.-691G>T, c.47C>T, c.995C>T |

Below is a table with the results of the sleep questionnaires carried out before supplementation and at the 20-day supplementation:

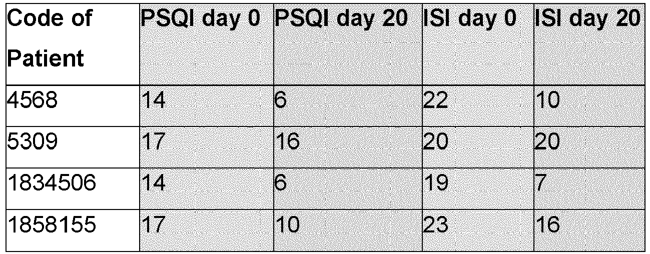

The scores are from 0 to 28 in the case of the ISI, and from 0 to 21 in the case of the PSQI. In both questionnaires, a higher score implies greater severity of insomnia (ISI) or worse sleep quality (PSQI). For further information about questionnaires, see Célyne H. Bastien, et al., Validation of the Insomnia Severity Index as an outcome measure for insomnia research. Sleep Medicine 2 (2001) 297-307, for ISI, and Daniel J. Buysse et al., The Pittsburgh Sleep Quality Index: A New Instrument for Psychiatric Practice and Research., Psychiatry Research 28, 193, 213, accepted Nov. 12, 1988, for PSQI.

In conclusion from the results, in the supplemented patients it can be observed that the 3 patients who have 3 or 4 heterozygous variants have experienced an improvement during the first days which, in 2 cases, has subsided after 20 days of supplementation. In the patient with only 1 heterozygous variant, no improvement was observed throughout the supplementation.

Finally, detailed comments regarding the 2 visits made by the 4 subjects who carried out the supplementation are shown.

### SUBJECT 4568

FIRST VISIT 10/18th/2023

### Demographic and anthropometric data

44 year old woman.

She reports a height of 163 cm and weight of 58kg (BMI = 1.8).

### Related to sleep

She reports chronic maintenance insomnia, with daily frequency. It presents daytime repercussions of lack of sleep (fatigue, lack of attention and memory, mood changes and headaches).

She goes to bed around 11:00 p.m. and wakes up at 7:00 a.m., with sleep latency of 5 minutes and total perceived daily sleep of 4 hours.

Through nocturnal polysomnography, sleep apnea and periodic leg movement disorder are ruled out.

### Relative to DAO deficit

She refers to symptoms associated with a DAO deficiency: digestive problems (irritable bowel) and allergies (rhinitis, urticaria).

A DAO genetic variant test is performed, with 3 heterozygous mutations:
- c. -691G>T (rs2052129)
- c. 47C>T (rs10156191)
- c. 995C>T (rs1049742)

### Medication

She is currently not taking sleeping medication.

### Sleep questionnaires

She conducts two sleep questionnaires in consultation: Insomnia Severity Index (ISI) and Pittsburgh Sleep Quality Index (PSQI).
- ISI score: **22**/28 → clinical insomnia (severe)
- PSQI score: **14**/21
   ∘ Component 1 (subjective sleep quality): 2/3
   ∘ Component 2 (sleep latency): 1/3
   ∘ Component 3 (sleep duration): 3/3
   ∘ Component 4 (habitual sleep efficiency): 3/3
   ∘ Component 5 (sleep disturbances): 2/3
   ∘ Component 6 (use of sleep medication): 0/3
   ∘ Component 7 (dysfunction during the day): 3/3

Starts with the indicated supplementation: 1 tablet, 3 times a day, 20 minutes before main meals.

### SECOND VISIT (20 DAYS OF SUPPLEMENTATION) 11/08th/2023

### Related to taking the supplement

She has completed all the treatment doses. She only had a problem with one day (she took a double dose), and another day the pill didn't come out.

### Related to sleep

With DAO she noticed improvement from the first week: deeper sleep, although 2-3 awakenings persisted with a frequency of less than 2 times a week.

She has brought her bedtime forward to 10:30 p.m. and gets up around 6:45 a.m. (6 hours of total average sleep). She reports a sleep latency of less than 15 minutes, and occasional episodes of nocturia. She falls asleep on the couch less often, when she is sleepy, she goes to bed.

Regarding daytime repercussions, she reports drowsiness (in a state of inactivity), fatigue, lack of concentration and lack of attention.

Observation: she reported many contractures and impingement of the suprascapular nerve, taking diazepam with a prescription for 5 days (started the day before the second DAO supplementation visit).

### Relative to DAO deficit

She reports an improvement in digestive problems (irritable bowel) that are reflected in less bloating and less diarrhea.

She reported seasonal allergic rhinitis worse than in other years, coinciding with taking DAO. 1 day she took antihistamine (desloratadine). This symptomatology stopped 3 days ago.

### Medication

Took 1 diazepam in the last week (due to muscle contracture).

### Sleep questionnaires

She conducts two sleep questionnaires in consultation: Insomnia Severity Index (ISI) and Pittsburgh Sleep Quality Index (PSQI).
- ISI score: **10**/28 → subclinical insomnia
- PSQI score: **6**/21
   ∘ Component 1 (subjective sleep quality): 1/3*
   ∘ Component 2 (sleep latency): 0/3*
   ∘ Component 3 (sleep duration): 1/3*
   ∘ Component 4 (usual sleep efficiency): 2/3*
   ∘ Component 5 (sleep disturbances): 1/3*
   ∘ Component 6 (use of sleep medication): 1/3
   ∘ Component 7 (dysfunction during the day): 0/3*
      *Marked with an asterisk are the PSQI components in which improvement is observed (for each component, 0 is the best score and 3 is the worst)

Continuous supplementation indicated: 1 tablet, 3 times a day, 20 minutes before main meals.

### Conclusion

The patient shows improvement in the quality and quantity of her sleep, reflected in the two questionnaires (PSQI and ISI) and in the clinical history.

### SUBJECT 5309

### FIRST VISIT 10/18th/2023

### Demographic and anthropometric data

66-year-old woman. She reports a height of 167cm and weight of 58kg (BMI = 20.8).

### Related to sleep

She reports chronic maintenance insomnia, with daily frequency. She presents daytime repercussions of lack of sleep (drowsiness, occasional fatigue, mood changes: sadness). She goes to bed around 11:00 p.m. and wakes up at 8:00 a.m., with a sleep latency of 10 minutes and total perceived daily sleep of 4.5 hours.

Through nocturnal polysomnography, sleep apnea and periodic leg movement disorder are ruled out.

### Relative to DAO deficit

She refers to symptoms associated with a DAO deficiency: asthma (she takes Spiriva Respimat daily). A DAO genetic variant test is performed, with **1 heterozygous mutation:**
- c. -691G>T (rs2052129)

### Medication

Currently taking:
- Melatonin (Circadin) daily
- Clonazepam (Rivotril) 2 times in the last week.

### Sleep questionnaires

She conducts two sleep questionnaires in consultation: Insomnia Severity Index (ISI) and Pittsburgh Sleep Quality Index (PSQI).
- ISI score: **20**/28 → clinical insomnia (moderate)
- PSQI score: **17**/21
   ∘ Component 1 (subjective sleep quality): 3/3
   ∘ Component 2 (sleep latency): 1/3
   ∘ Component 3 (sleep duration): 3/3
   ∘ Component 4 (habitual sleep efficiency): 3/3
   ∘ Component 5 (sleep disturbances): 2/3
   ∘ Component 6 (use of sleep medication): 3/3
   ∘ Component 7 (daytime dysfunction): 2/3

Start indicated supplementation: 1 tablet, 3 times a day, 20 minutes before main meals.

### SECOND VISIT (20 DAYS OF SUPPLEMENTATION) 11/08th/2023

### Related to taking the supplement

She has completed all the treatment doses.

### Related to sleep

She reports no improvement in sleep, with 3-5 nocturnal awakenings and 5 hours of total perceived sleep. After falling asleep (latency of 16-30 minutes), the first sleep is 1.5-3 hours long. There are days when she has spent 11 hours in bed.

She comments that despite clonazepam (3 doses in the last 15 days) she cannot continue sleeping.

She has some nightmares and on one occasion a throbbing sensation in her head (it had not happened before).

She presents daytime repercussions (drowsiness, fatigue, cognitive deficit, lack of concentration, lack of attention and memory, mood changes and headaches). If she sleeps little time, she feels more depressed.

### Relative to DAO deficit

She does not report worsening of asthma or congestion.

### Medication

Currently taking:
- Melatonin (Circadin) daily
- Clonazepam (Rivotril) 3 times in the last 15 days.

### Sleep questionnaires

She conducts two sleep questionnaires in consultation: Insomnia Severity Index (ISI) and Pittsburgh Sleep Quality Index (PSQI).
- ISI score: 2**0/**28 → clinical insomnia (moderate)
- PSQI score: **16**/21
   ∘ Component 1 (subjective sleep quality): 3/3
   ∘ Component 2 (sleep latency): 2/3
   ∘ Component 3 (sleep duration): 2/3*
   ∘ Component 4 (habitual sleep efficiency): 3/3
   ∘ Component 5 (sleep disturbances): 2/3
   ∘ Component 6 (use of sleeping medication): 2/3*
   ∘ Component 7 (daytime dysfunction): 2/3
      *PSQI components in which improvement is observed are marked with an asterisk Continuous supplementation indicated: 1 tablet, 3 times a day, 20 minutes before main meals.

### Conclusion

The patient shows no improvement in the quality or quantity of her sleep. The sleep questionnaires (ISI and PSQI) reflect a score similar to the initial score. Improvement is also ruled out in the clinical history.

### SUBJECT 1834506

### FIRST VISIT 10/18th/2023

### Demographic and anthropometric data

61-year-old woman. She reports a height of 170cm and weight of 59kg (BMI = 20.4).

### Related to sleep

She reports chronic maintenance insomnia, with daily frequency. She presents daytime repercussions of lack of sleep (mild drowsiness).

She goes to bed around 9:00 p.m. and wakes up at 5:00 a.m., with sleep latency of 10 minutes and total perceived daily sleep of 4 hours.

### Relative to DAO deficit

She does not report symptoms associated with a DAO deficiency. She reports hypothyroidism while being treated with Eutirox.

A DAO genetic variant test is performed, with **4 heterozygous mutations:**
- c. -691G>T (rs2052129)
- c. 47C>T (rs10156191)
- c. 995C>T (rs1049742)
- c. 1990C>G (rs1049793)

### Medication

She currently takes melatonin 1.9 mg (she reports 1 intake in the last 7 days).

### Sleep questionnaires

She conducts two sleep questionnaires in consultation: Insomnia Severity Index (ISI) and Pittsburgh Sleep Quality Index (PSQI).
- ISI score: **19**/28 → clinical insomnia (moderate)
- PSQI score: **14**/21
   ∘ Component 1 (subjective sleep quality): 3/3
   ∘ Component 2 (sleep latency): 0/3
   ∘ Component 3 (sleep duration): 3/3
   ∘ Component 4 (habitual sleep efficiency): 3/3
   ∘ Component 5 (sleep disturbances): 1/3
   ∘ Component 6 (use of sleep medication): 3/3
   ∘ Component 7 (daytime dysfunction): 1/3

Start indicated supplementation: 1 tablet, 3 times a day, 20 minutes before main meals.

### SECOND VISIT (20 DAYS OF SUPPLEMENTATION) 09/11th/2023

### Related to taking the supplement

During the first two weeks, she took her tablets regularly (1 tablet 20 minutes before the 3 main meals). From the third week onwards, she took the three tablets before dinner (the first day due to an oversight, and then she continued to notice an improvement in the effect). She may have forgotten to take some tablets (the dispenser is not empty).

### Related to sleep

The first week (taking DAO 3 times a day) she reported sleeping an hour more, but "much worse." She comments on the events of many nightmares, which she never had before, and a lot of sleep interruption. It may have had something to do with alcohol intake. If she goes out to dinner or lunch, she drinks wine ("at least 2 glasses"). There have been no emotional problems, large meals or changes in diet that could justify the appearance of nightmares. She does not report an increase in commitments or eating out (she believes she went out to dinner 1 or 2 times during that week).

The second week she added 1 occasional dose of melatonin (1mg), and continued with many sleep interruptions.

The third week she took 3 doses at a time (before dinner). Since the first day she made this change she has been sleeping 6 hours and does have some awakenings (she comments that she has not slept that much in 20 years). She has taken melatonin occasionally during the week. The last night without taking melatonin she slept 7 hours, and she does not remember dreaming.

The sleep schedule for the last week has been from 10-11 p.m. to 6 a.m.

### Sleep questionnaires

She conducts two sleep questionnaires in consultation: Insomnia Severity Index (ISI) and Pittsburgh Sleep Quality Index (PSQI). **She answers them based on her condition on the last week of treatment.**
- ISI score: **7**/28 → non-clinically significant insomnia
- PSQI score: **6**/21
   ∘ Component 1 (subjective sleep quality): 1/3*
   ∘ Component 2 (sleep latency): 0/3
   ∘ Component 3 (sleep duration): 1/3*
   ∘ Component 4 (usual sleep efficiency): 1/3*
   ∘ Component 5 (sleep disturbances): 1/3
   ∘ Component 6 (use of sleeping medication): 2/3*
   ∘ Component 7 (dysfunction during the day): 0/3*

*PSQI components in which improvement is observed are marked with an asterisk Continuous supplementation indicated: 1 tablet, 3 times a day, 20 minutes before main meals.

### Conclusion

The patient shows improvement in the quality and quantity of her sleep, reflected in the two questionnaires (PSQI and ISI) and in the clinical history, especially when taking 3 doses of DAO in a row.

### SUBJECT 1858155

### FIRST VISIT 10/18th/2023

### Demographic and anthropometric data

42-year-old man. She reports a height of 170cm and weight of 88.5kg (BMI = 30.6).

### Related to sleep

She reports chronic insomnia due to sleep, maintenance and early awakening, with daily frequency. It presents daytime repercussions of lack of sleep (drowsiness, lack of concentration, lack of attention and memory, mood changes and headaches).

She goes to bed around 10:00 p.m. and wakes up at 6:30 a.m., with sleep latency of 90-120 minutes and total perceived daily sleep of 5 hours.

Through nocturnal polysomnography, mild sleep apnea (AHI =8.6/h) is detected and periodic leg movement disorder is ruled out.

### Relative to DAO deficit

She refers to symptoms associated with a DAO deficiency: migraine, anxiety disorder, digestive problems (hiatal hernia) and allergies (pollen, mites and banana trees).

A DAO genetic variant test is performed, with **3 heterozygous mutations:**
- c. -691G>T (rs2052129)
- c. 47C>T (rs10156191)
- c. 995C>T (rs1049742)

### Medication

She currently takes diazepam occasionally (she reports 2 doses in the last 7 days).

### Sleep questionnaires

She conducts two sleep questionnaires in consultation: Insomnia Severity Index (ISI) and Pittsburgh Sleep Quality Index (PSQI).
- ISI score: **23**/28 → clinical insomnia (severe)
- PSQI score: **17**/21 → poor sleep quality
   ∘ Component 1 (subjective sleep quality): 2/3
   ∘ Component 2 (sleep latency): 3/3
   ∘ Component 3 (sleep duration): 2/3
   ∘ Component 4 (habitual sleep efficiency): 3/3
   ∘ Component 5 (sleep disturbances): 2/3
   ∘ Component 6 (use of sleep medication): 2/3
   ∘ Component 7 (dysfunction during the day): 3/3

Start indicated supplementation: 1 tablet, 3 times a day, 20 minutes before main meals.

### SECOND VISIT (20 DAYS OF SUPPLEMENTATION) 11/08th/2023

### Related to taking the supplement

She has completed all the treatment doses, on occasion he reports forgetting the 20-minute regimen before and has taken it during and after meals.

### Related to sleep

She falls asleep better (she even mentions that she has not slept this well since she was a teenager) and the awakenings continue to occur (frequency x3 times) but with easy conciliation again. On one occasion she took a 2-hour nap and fell asleep again at night. Regarding the daytime repercussions, fatigue has improved and somewhat better drowsiness has been noted. Before the first visit, she reported that she was very sleepy and even fell asleep on the subway.

### Relative to DAO deficit

She has not had headaches, but comments that this happens sporadically. She has improved digestive problems, but says it is due to improved meals.

Observation: At the same time, she has had a cold for more than 1 week. She has not taken antihistamines, if she is very congested she can use it on a specific day with prior communication. She mentions that a cold affects questions 5d and 5e of the PSQI questionnaire ("not being able to breathe well" and "coughing or snoring loudly").

### Medication

She has not taken sleeping medication in the last 3 weeks.

### Sleep questionnaires

She conducts two sleep questionnaires in consultation: Insomnia Severity Index (ISI) and Pittsburgh Sleep Quality Index (PSQI).
- ISI score: **16**/28 → clinical insomnia (moderate)
- PSQI score: **10**/21 → poor sleep quality
   ∘ Component 1 (subjective sleep quality): 1/3*
   ∘ Component 2 (sleep latency): 2/3*
   ∘ Component 3 (sleep duration): 1/3*
   ∘ Component 4 (usual sleep efficiency): 2/3*
   ∘ Component 5 (sleep disturbances): 2/3
   ∘ Component 6 (use of sleep medication): 0/3*
   ∘ Component 7 (dysfunction during the day): 2/3*
      *PSQI components in which improvement is observed are marked with an asterisk Continuous supplementation indicated: 1 tablet, 3 times a day, 20 minutes before main meals.

### Conclusion

The patient shows improvement in the quality and quantity of her sleep, reflected in the two questionnaires (PSQI and ISI) and in the clinical history.

## Claims

1. Diamine oxidase (DAO) for use in the treatment and/or prevention of sleep and circadian rhythm disorders, and cognitive impairment associated thereto.

2. The diamine oxidase for use according to claim 1, wherein said sleep and circadian rhythm disorders are insomnia and/or abnormal dreams.

3. The diamine oxidase for use according to claim 1 or 2, wherein said DAO is combined with at least one another active ingredient.

4. The diamine oxidase for use according to claim 3, wherein said at least one another active ingredient is a botanical extract selected from Crocus sativus L., Melissa officinalis, Melissa officinalis folium, Avena sativum, Crataegus laevigata, Lavandula officinalis flos, Lavandula angustifolia, Melilotus officinalis, Papaver rhoeas, Valeriana officinalis, Valeriana officinalis radix, Bigaradier feuille, Valerian- Humulus lupulus combination, Tilia species flos, Tilia platyphyllos, Aloysia triphylla, Anethum graveolens, Centaurium erythrea, Galium odoratum, Humulus lupulus, Humulus lupulus strobuli, Eschscholtzia californica, Asparagus racemosus root, Convolvulus pluricaulis, combination of valerian root, lemon balm leaves and chamomile flowers, Sida cordifolia, Withania somnifera, Bitter orange, Hypericum perforatum-Herba hyperici plant-St. John Wort, Salix alba-Buds-White Willow, Thuja orientalis-seeds-Arbor vitae, Passiflora incarnata herba, Melilotus officinalis herba, Majorana hortensis herba, Crataegus monogyna folium cum flore, Matricaria chamomilla flos, Magnesium lactate, Hippophae rhamnoides, sea-buckthorns, fruit vitamin B6-PVP-aerosil-starch-tablettose-avicel-stearic acid, Mentha piperita, Ballota nigra L., xanthohumol enriched hop extract, Hop extract containing xanthohumol, xanthohumol, Hop extract, Artemisia dracunculus, Grindelia robusta nutt., Marrubium vulgare I., Ruta graveolens I., Tabebuia avellanedae, Passiflora incarnata, passion flower, alcohol water Pi-nipagin-nipasol, Ziziphi Spinosae, Panax ginseng, Panax quinquefolius, Poria cocos, Polygala tenuifolia, Ganoderma lucidum, Schisandra chinensis, Platycladus orientalis, Acanthopanax senticosus, Fallopia multifloraVeronica officinalis, Calluna vulgaris, Filipendula ulmaria, Bacopa montiera, Basilic, Cassia italica f.w. andr., Epilobium angustifolium I., Primula veris I. em.heids, Tanacetum parthenium, and a combination thereof.

5. The diamine oxidase for use according to claim 3 or 4, wherein said at least one another active ingredient is a pharmaceutical drug for treating sleep and circadian rhythm disorders selected from sedating antidepressants, sedating antipsychotics, benzodiazepine receptor agonists, non-benzodiazepine benzodiazepine receptor agonists (nBBRAs), barbiturates, inhibitors of orexin and hypocretin, doxepin, melatonin, agonists of melatonin receptors, and a combination thereof.

6. The diamine oxidase for use according to any of the preceding claims, wherein said diamine oxidase is included in a pharmaceutical composition.

7. The diamine oxidase for use according to claim 6, wherein said pharmaceutical composition further comprises a pharmaceutically acceptable vehicle, adjuvant, diluent or excipient, and optionally at least another active ingredient.

8. The diamine oxidase for use according to any of the preceding claims, wherein said prevention and/or treatment comprises topically, transdermally or orally administering said diamine oxidase or the pharmaceutical composition comprising thereof.

9. The diamine oxidase for use according to any of the preceding claims, wherein said diamine oxidase or the pharmaceutical composition comprising thereof is in a dosage form selected from powder, tablet, capsule, microcapsule, minicapsule, nanocapsule, sachet, liposome, drink, liquid in spray, cream, lotion, ointment, patch, and liposomes.

10. The diamine oxidase for use according to any of the claims 1-5, wherein said diamine oxidase is included in a functional food or a dietary supplement.

11. The diamine oxidase for use according to any of the claims 5 to 10, wherein the pharmaceutical composition, the dietary supplement or the functional food is administered before or with meals and/or once per day

12. The diamine oxidase for use according to claim 11, wherein the pharmaceutical composition, the dietary supplement or the functional food is administered once per day before going to sleep.

13. The diamine oxidase for use according to any of the claims 1-6, wherein said diamine oxidase is included in a cosmetic formulation.

14. The diamine oxidase for use according to any of the claims 1-6, wherein said diamine oxidase is a medical device or is included in powder, semi-solid, semi-liquid or liquid form in a delivery system which is a medical device.
